(19) Europäisches Patentamt
European Patent Office
Office européen des brevets

(11) Numéro de publication : **0 061 406**
**B1**

(12) # FASCICULE DE BREVET EUROPÉEN

(45) Date de publication du fascicule du brevet : **13.06.84**

(51) Int. Cl.³ : **C 07 C103/76**, A 61 K 31/165

(21) Numéro de dépôt : **82400520.1**

(22) Date de dépôt : **23.03.82**

(54) **Dérivés d'acide benzamido-alkyl-hydroxamique, procédé de préparation et composition thérapeutique.**

(30) Priorité : **25.03.81 FR 8106017**

(43) Date de publication de la demande : **29.09.82 Bulletin 82/39**

(45) Mention de la délivrance du brevet : **13.06.84 Bulletin 84/24**

(84) Etats contractants désignés : **AT BE CH DE FR GB IT LI LU NL SE**

(56) Documents cités : **FR-A- 2 345 430**

(73) Titulaire : **LABORATOIRE L. LAFON Société anonyme dite: 1 rue Georges Médéric F-94701 Maisons-Alfort (FR)**

(72) Inventeur : **Lafon, Louis 5 rue de l'Alboni F-75016 Paris (FR)**

(74) Mandataire : **Clisci, Serge et al CABINET BEAU DE LOMENIE 55 rue d'Amsterdam F-75008 Paris (FR)**

**EP 0 061 406 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

## Description

La présente invention concerne, en tant que produits industriels nouveaux, des dérivés appartenant à la famille des acides benzamido-alkyl-hydroxamiques. Elle concerne également leur procédé de préparation et des compositions thérapeutiques.

On connaît du brevet belge n° 852 738 le chlorhydrate de l'acide 2-(4-aminobenzamido)-acétohydroxamique qui a pour n° de code CRL 40473 (voir exemple 18 dudit brevet belge). On vient de trouver de façon surprenante que les dérivés de formule I ci-après, qui sont structurellement différents du produit connu précité, présentent des propriétés très intéressantes sur le plan thérapeutique, notamment en ce qui concerne les propriétés anti-agressives.

Selon l'invention, on préconise donc un nouveau dérivé d'acide benzamido-alkyl-hydroxamique caractérisé en ce qu'il est choisi parmi l'ensemble constitué par

(i) les acides 3-(aminobenzamido)-propionhydroxamiques de formule générale

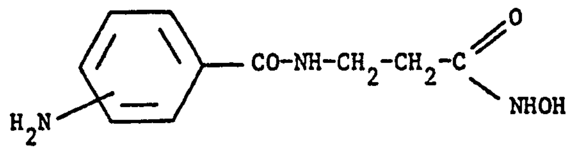

(I)

et

(ii) leurs sels d'addition avec les acides.

L'invention vise donc les acides o-, m- et p-amino-benzamido-propionhydroxamiques et leurs sels, les produits préférés étant l'acide 3-(3-amino-benzamido)-propionhydroxamique et ses sels d'addition, notamment le chlorhydrate.

Par sels d'addition, on entend ici les sels d'addition d'acides obtenus par réaction d'une base libre de formule I avec un acide minéral ou organique. Parmi les acides utilisables pour salifier les bases de formule I, on peut notamment citer les acides chlorhydrique, bromhydrique, nitrique, sulfurique, acétique, propionique, oxalique, fumarique, maléique, succinique, benzoïque, cinnamique, mandélique, citrique, malique, tartrique, p-toluène-sulfonique et méthanesulfonique.

Les composés de formule I peuvent être préparés selon une méthode connue par application de mécanismes réactionnels classiques. On peut, par exemple, les préparer selon les modalités opératoires de l'exemple 18 du brevet belge précité. Le procédé que l'on préconise selon l'invention pour préparer un acide 3-(amino-benzamido)-propionhydroxamique est caractérisé en ce qu'on soumet à une réaction d'hydrogénation catalytique un acide 3-(nitrobenzamido)-propionhydroxamique de formule

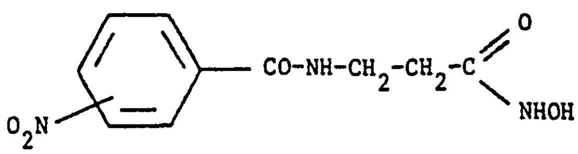

(II)

au moyen d'un catalyseur constitué par du palladium sur charbon.

Les composés de formule I et leurs sels d'addition sont utiles en thérapeutique sur le SNC, notamment en tant qu'agents sédatifs.

Selon l'invention, on préconise enfin une composition thérapeutique qui est caractérisée en ce qu'elle renferme, en association avec un excipient physiologiquement acceptable, au moins un composé de formule I ou l'un de ses sels non toxiques. Bien entendu, une telle composition renfermera une quantité pharmaceutiquement efficace d'ingrédient actif.

D'autres avantages et caractéristiques de l'invention seront mieux compris à la lecture qui va suivre d'exemples de préparation nullement limitatifs.

### Préparation I

Obtention du chlorhydrate de l'acide 3-(3-aminobenzamido)-propionhydroxamique

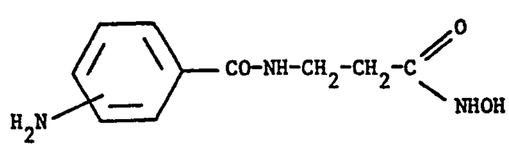

HCl

(n° de code CRL 40816)

a) m-Nitrobenzamidopropionate d'éthyle

On coule goutte à goutte à reflux, une solution de 18,55 g (0,1 mole) de chlorure de m-nitrobenzoyle dans 100 ml de benzène à une solution de 15,35 g (0,1 mole) de chlorhydrate d'aminopropionate d'éthyle dans 100 ml de benzène. On maintient le reflux pendant environ 6 heures, lave la phase benzénique à l'eau, avec une solution de bicarbonate dilué, puis avec HCl dilué et de nouveau à l'eau. On sèche sur $Na_2SO_4$, et après évaporation recueille 24,4 g (rendement : 91 %) de m-nitrobenzamido-propionate d'éthyle qui se présente sous la forme d'une huile de couleur jaune-orangé.

b) Acide m-nitrobenzamidopropionhydroxamique

On prépare une solution de méthylate de sodium avec 4,22 g (0,183 at-g) de Na dans 150 ml de méthanol anhydre et ajoute à froid, une quantité de 6,37 g (0,091 7 mole) de chlorhydrate d'hydroxylamine en solution dans 120 ml de méthanol anhydre. On élimine par filtration le chlorure de sodium formé et ajoute au filtrat 24,4 g (0,091 7 mole) de m-nitrobenzamidopropionate d'éthyle. Après une nuit en contact à température ambiante, on évapore à sec sous vide, reprend avec suffisamment d'eau de façon à dissoudre le sel de sodium et acidifie avec HCl concentré ($d_4^{15}$ = 1,19). On essore le précipité ainsi obtenu et lave à l'eau. On obtient après recristallisation dans l'eau 17,1 g (rendement : 74 %) d'acide m-nitrobenzamidopropionhydroxamique.

c) CRL 40816

On procède à une hydrogénation catalytique, au reflux pendant 5 heures, à partir d'un mélange de 16, 45 g (0,065 mole) d'acide m-nitrobenzamidopropionhydroxamique, 21 ml de cyclohexène, 2,2 g de Pd/charbon à 10 % et 120 ml d'éthanol anhydre. On filtre le Pd/charbon, évapore à sec sous vide et reprend à l'éthanol. On précipite le chlorhydrate au moyen d'éthanol chlorhydrique, filtre puis lave le précipité avec un peu d'éther. On recueille après recristallisation dans le mélange eau-éthanol (10 : 90) v/v 10,7 g (rendement : 64 %) de CRL 40816 qui se présente sous la forme d'une poudre légèrement rose, insoluble dans les alcools, l'acétone, l'éther et très soluble dans l'eau. F = 206 °C.

En procédant comme indiqué ci-dessus et en remplaçant le chlorure de m-nitrobenzoyle par les chlorures de o- et p-nitrobenzoyle, on obtient respectivement les acides 3-(2-aminobenzamido)- et 3-(4-aminobenzamido)-propionhydroxamiques.

On a résumé ci-après les résultats des essais qui ont été entrepris avec le CRL 40816. Dans ces essais il a été administré par voie intrapéritonéale, sous un volume de 20 ml/kg chez la souris mâle, et sous un volume de 5 mg/kg chez le rat mâle.

1. Toxicité

La dose maximale non mortelle (DL-O) par voie I.P. chez la souris mâle est supérieure à 1 024 mg/kg.

2. Comportement global

Des lots de 10 animaux par dose sont observés avant, puis 15 minutes, 30 minutes, 1 heure, 2 heures, 3 heures et 24 heures après administration I.P. de CRL 40816. On constate a) chez la souris qu'à la dose de 512 mg/kg le CRL 40816 provoque

(i) une sédation avec diminution de la réactivité au toucher pendant 3 heures,
(ii) une hypothermie modérée (– 1,3 °C) pendant 2 heures, et
(iii) une respiration déprimée pendant 3 heures ; et b) chez le rat qu'à partir de la dose de 256 mg/kg, le CRL 40816 entraîne
(i) une diminution de la réactivité au toucher et du tonus musculaire pendant 3 heures, et
(ii) une mydriase pendant 3 heures.

3. Action sur le SNC

a) Interaction avec l'apomorphine

— Chez la souris :

Une demi-heure après l'administration du CRL 40816, des lots de 6 souris reçoivent une injection sous-cutanée d'apomorphine à la dose de 1 ou 16 mg/kg. On observe ce qui suit : aux faibles doses, le CRL 40816 ne modifie pas l'hypothermie, l'attitude de verticalisation et les stéréotypies induites par l'apomorphine chez la souris ; à la dose de 32 mg/kg, le CRL 40816 semblerait induire un antagonisme discret de l'hypothermie induite par la plus faible dose d'apomorphine ; et à dose plus élevée (512 mg/kg) on constate une diminution sensible de la température à la suite de l'injection de CRL 40816.

— Chez le rat :

Des lots de 6 rats reçoivent le CRL 40816 une demi-heure avant l'injection sous-cutanée d'apomorphine à la dose de 0,5 mg/kg. On observe que le CRL 40816 ne modifie pas le comportement stéréotypé provoqué par l'apomorphine chez le rat.

b) Interaction avec l'amphétamine

Une demi-heure après l'administration du CRL 40816, des lots de 6 rats reçoivent une injection intrapéritonéale de 2 mg/kg d'amphétamine. On observe qu'à forte dose (256 mg/kg), le CRL 40816 entraîne une diminution modérée de l'intensité des stéréotypies amphétaminiques.

c) Interaction avec la réserpine

Quatre heures après l'injection intrapéritonéale de 2,5 mg/kg de réserpine, des lots de 6 souris reçoivent le CRL 40816. On observe que le CRL 40816 ne modifie pas l'hypothermie ni le ptosis provoqués par la réserpine.

d) Interaction avec l'oxotrémorine

L'oxotrémorine (0,5 mg/kg — I.P.) est injectée à des lots de 6 souris une demi-heure après l'administration de CRL 40816.

Action sur la température

A la dose de 32 mg/kg, le CRL 40816 semble s'opposer modérément et tardivement à l'action hypothermisante de l'oxotrémorine. Par contre, à forte dose (512 mg/kg), le CRL 40816 exerce un effet hypothermisant et aggrave la baisse de température due à l'oxotrémorine.

Action sur les tremblements

Le CRL 40816 laisse inchangés les tremblements produits par l'oxotrémorine.

Action sur les symptômes cholinergiques périphériques

Les signes de stimulation cholinergique périphérique (salivation, lacrymation, défécation), qui apparaissent à la suite d'une injection d'oxotrémorine, ne sont pas modifiés par le CRL 40816.

e) Action sur le test des quatre plaques, la traction et l'électrochoc

Des lots de 10 souris sont étudiés une demi-heure après l'administration de CRL 40816.
On observe que le CRL 40816 n'entraîne pas d'augmentation du nombre de passages punis ; il ne provoque pas d'incapacité motrice majeure et ne modifie pas les effets convulsivants et létaux de l'électrochoc.

f) Action sur la motilité spontanée

Une demi-heure après avoir reçu le CRL 40816, les souris (6 par dose, 12 témoins) sont placées en actimètre où leur motilité est enregistrée pendant 30 minutes.
A forte dose (512 mg/kg) on constate que le CRL 40816 entraîne une hypomotilité modérée.

g) Action sur l'agressivité intergroupes (étude comparative)

Après avoir séjourné durant 3 semaines de part et d'autre d'une cloison opaque séparant leur cage par le milieu, des groupes de 3 souris mâles (chaque souris pesant environ 20 g) reçoivent les produits à tester (CRL 40816 et CRL 40473) par voie I.P. en solution dans de l'eau distillée, à raison de trois cages par produit et par dose et de six cages pour les animaux témoins ne recevant que de l'eau distillée par voie I.P. Une demi-heure plus tard, les deux groupes d'une même cage sont réunis et on note le nombre de combats qui surviennent en 10 minutes. Les résultats sont consignés dans le tableau I qui suit et démontrent que le CRL 40816 selon l'invention (i) diminue fortement le nombre de combats à la dose de 128 mg/kg, (ii) supprime totalement les combats à la dose de 512 mg/kg, et (iii) possède un effet antiagressif bénéfique nettement supérieur à celui du CRL 40473 selon l'exemple 18 du brevet belge précité.

Tableau I

| Produit | dose (mg/kg) | nombre de combats par souris | diminution du nombre de combats par rapport aux témoins |
|---|---|---|---|
| témoins | - | 3,13 | 0 % |
| CRL 40816 | 128 | 1,44 | 54 % |
| CRL 40816 | 512 | 0 | 100 % |
| CRL 40473 | 128 | 2,25 | 28 % |
| CRL 40473 | 512 | 1,82 | 41 % |

h) Action vis-à-vis de quelques comportements perturbés par divers agents

Motilité réduite par habituation à l'enceinte

Après 18 heures de séjour dans les actimètres, les souris (6 par dose, 12 témoins) reçoivent le CRL 40816. Elles sont aussitôt replacées dans leurs enceintes respectives et, une demi-heure plus tard, on enregistre leur motilité pendant 30 minutes.

On observe que le CRL 40816 ne provoque pas, d'une manière générale, de reprise de l'activité chez la souris habituée à son enceinte.

Motilité réduite par agression hypoxique

Une demi-heure après avoir reçu le CRL 40816, les souris (10 par dose, 20 témoins) sont soumises à une anoxie hypobare aiguë [dépression de 600 mm Hg (soit environ 8 × $10^4$ pascals) en 90 secondes, détente de 45 secondes], puis elles sont placées en actimètre où leur motilité est enregistrée pendant 10 minutes.

On observe que le CRL 40816 n'entraîne pas d'amélioration de la récupération motrice chez les souris dont la motilité a été déprimée à la suite d'un séjour bref dans une enceinte à pression réduite.

Anoxie asphyxique

Une demi-heure après l'administration de CRL 40816, des lots de 10 souris reçoivent une injection intrapéritonéale de 32 mg/kg de triiodoéthylate de gallamine.

On note qu'à la plus forte dose utilisée (512 mg/kg), le CRL 40816 retarde l'apparition des convulsions et de la mort consécutives à une anoxie asphysique provoquée par un curarisant tel que le triiodoéthylate de gallamine.

En conclusion, il résulte de l'ensemble des essais sur le SNC, que le CRL 40816 présente des propriétés sédatives à forte dose : hyporéactivité, hypomotilité et hypothermie, qui permettent d'expliquer la résistance à l'anoxie, d'une part, et la diminution de l'agressivité et des stéréotypies amphétaminiques, d'autre part. De plus les résultats observés à 32 mg/kg (l'antagonisme partiel de l'hypothermie induite par l'oxotrémorine, par la faible dose d'apomorphine, l'augmentation modérée du nombre des passages punis au test des quatre plaques et la légère amélioration de la récupération motrice après hypoxie hypobare) permettent de différencier le CRL 40816 des produits décrits précédemment.

En clinique, le CRL 40816 s'est révélé chez l'homme, sous forme comprimés ou gélules renfermant chacun 200 mg de principe actif, à raison de 2 comprimés ou gélules par jour, un excellent antidépresseur.

**Revendications** (pour les Etats contractants : BE, CH, DE, FR, GB, IT, LI, LU, NL, SE)

1. Nouveau dérivé d'acide benzamido-alkyl-hydroxamique, utile notamment en thérapeutique, caractérisé en ce qu'il est choisi parmi l'ensemble constitué par

(i) les acides 3-(aminobenzamido)-propionhydroxamiques de formule générale

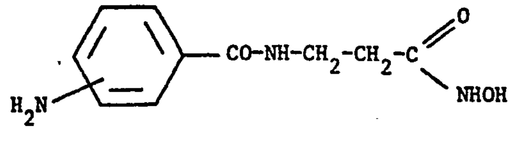

(I)

et

(ii) leurs sels d'addition avec les acides.

2. Acide 3-(3-aminobenzamido)-propionhydroxamique et ses sels d'addition avec les acides.

3. Acide 3-(2-aminobenzamido)-propionhydroxamique et ses sels d'addition avec les acides.

4. Acide 3-(4-aminobenzamido)-propionhydroxamique et ses sels d'addition avec les acides.

5. Composition thérapeutique, caractérisée en ce qu'elle renferme en association avec un excipient physiologiquement acceptable au moins un composé de formule I selon la revendication 1 ou l'un de ses sels d'addition d'acide non toxiques.

6. Procédé de préparation d'un composé de formule I selon la revendication 1, caractérisé en ce que l'on soumet à une réaction d'hydrogénation catalytique d'un acide 3-(nitrobenzamido)-propionhydroxamique de formule

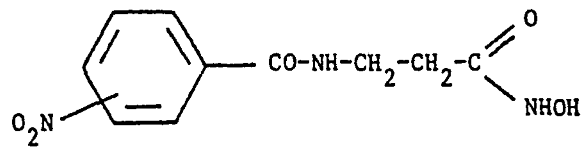

(II)

au moyen d'un catalyseur constitué par du palladium sur charbon.

**Revendication** (pour l'Etat contractant AT)

1. Procédé de préparation d'un acide 3-(aminobenzamido)-propionhydroxamique de formule générale

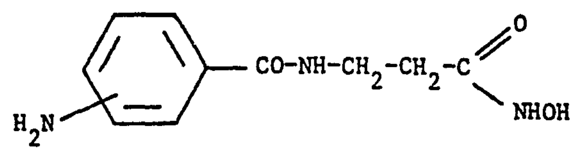

(I)

et de ses sels d'addition d'acide, ledit procédé étant caractérisé en ce que l'on soumet à une hydrogénation catalytique un acide 3-(nitrobenzamido)-propionhydroxamique de formule

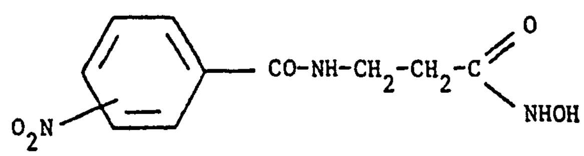

(II)

au moyen d'un catalyseur constitué par du palladium sur charbon.

**Claims** (for the Contracting States : BE, CH, DE, FR, GB, IT, LI, LU, NL, SE)

1. A benzamido-alkyl-hydroxamic acid derivative, which is useful in therapeutics, characterised in that it is selected from the group consisting of

(i) 3-(aminobenzamido)-propionhydroxamic acids of the general formula

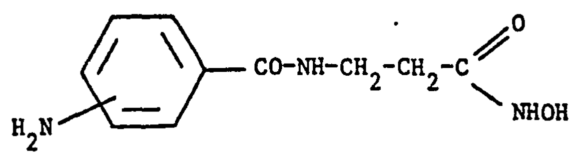

(I)

and

(ii) acid addition salts thereof.

2. 3-(3-Aminobenzamido)-propionhydroxamic acid and its acid addition salts.

3. 3-(2-Aminobenzamido)-propionhydroxamic acid and its acid addition salts.

4. 3-(4-Aminobenzamido)-propionhydroxamic acid and its acid addition salts.

5. A therapeutic composition, characterised in that it contains, in association with a physiologically acceptable excipient, at least a compound of formula I according to claim 1 or one of its non-toxic acid addition salts.

6. A method for preparing a compound of formula I according to claim 1, characterised in that a 3-(nitrobenzamido)-propionhydroxamic acid of the formula

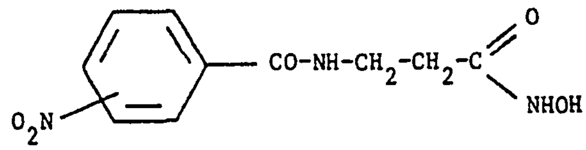

(II)

is subjected to a catalytic hydrogenation reaction with a Pd/C catalysator.

**Claim** (for the Contracting State AT)

1. A method for preparing a 3-(aminobenzamido)-propionhydroxamic acid of the general formula

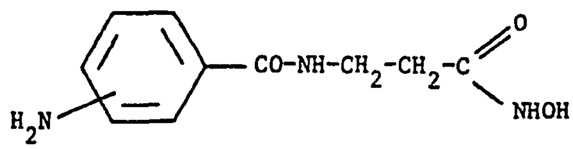

(I)

and its acid addition salts, said method being characterised in that a 3-(nitrobenzamido)-propionhydroxamic acid of the formula

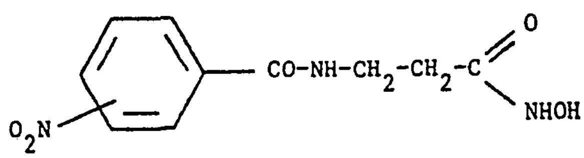

(II)

is subjected to a catalytic hydrogenation reaction with a Pd/C catalysator.

**Ansprüche** (für die Vertragsstaaten : BE, CH, DE, FR, GB, IT, LI, LU, NL, SE)

1. Derivat von Benzamido-alkylhydroxamsäure, das insbesondere in der Therapie geeignet ist, dadurch gekennzeichnet, dass es aus der Gruppierung, die aus

(i) den 3-(Aminobenzamido)-propionhydroxamsäuren der allgemeinen Formel

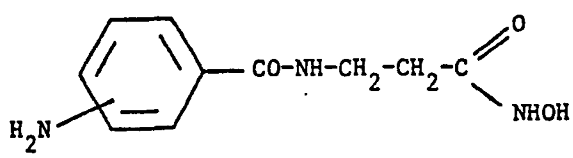

(I)

und

(ii) deren Säureadditionssalzen besteht, gewählt wird.

2. 3-(3-Aminobenzamido)-propionhydroxamsäure und ihre Säureadditionssalze.

3. 3-(2-Aminobenzamido)-propionhydroxamsäure und ihre Säureadditionssalze.

4. 3-(4-Aminobenzamido)-propionhydroxamsäure und ihre Säureadditionssalze.

5. Therapeutische Zusammensetzung, dadurch gekennzeichnet, dass sie zusammen mit einem physiologisch verträglichen Hilfstoff mindestens eine Verbindung der Formel I gemäss Anspruch 1 oder eines ihrer nicht toxischen Säureadditionssalze enthält.

6. Verfahren zur Herstellung einer Verbindung der Formel I gemäss Anspruch 1, dadurch gekennzeichnet, dass man eine 3-(Nitrobenzamido)-propionhydroxamsäure der Formel

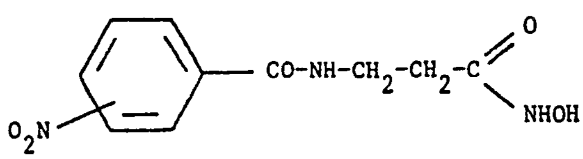

(II)

durch einen Pd/C Katalysator katalytisch hydrogeniert.

**Anspruch** (für den Vertragsstaat AT)

1. Verfahren zur Herstellung einer 3-(Aminobenzamido)-propionhydroxamsäure der Formel

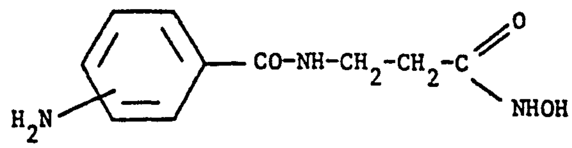

(I)

und ihrer Säureadditionssalze, dadurch gekennzeichnet, dass man eine 3-(Nitrobenzamido)-propionhydroxamsäure der Formel

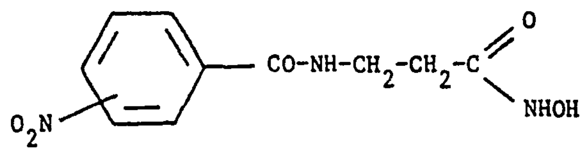

(II)

durch einen Pd/C Katalysator katalytisch hydrogeniert.